# EUROPEAN PATENT SPECIFICATION

(11) **EP 3 541 283 B1**
(45) Date of publication and mention of the grant of the patent: **11.08.2021**
(21) Application number: 17809203.7
(22) Date of filing: 13.11.2017
(51) Int. Cl.: A61B 5/15

(54) **A CORD BLOOD COLLECTION DEVICE**
VORRICHTUNG ZUR ENTNAHME VON NABELSCHNURBLUT
DISPOSITIF DE COLLECTE DE SANG OMBILICAL

(30) Priority: 18.11.2016 EP 16199701
(43) Date of publication of application: 25.09.2019
(73) Proprietor: The Hospital for the Relief of Poor-Lying-In Women, Dublin (Commonly called the Rotunda Hospital), Dublin 1 (IE)
(72) Inventor: CORBETT, Les, Bray A98 EF86 (IE)
(74) Representative: Purdylucey Intellectual Property
(86) International application number: PCT/EP2017/079092
(87) International publication number: WO 2018/091421

(56) References cited:
- US-A- 6 102 871
- US-A1- 2003 220 601
- US-B2- 8 486 034

## Description

### Field of the Invention

The invention relates to an umbilical cord blood collection device. In particular, this invention relates to a device for collecting cord blood for delivery to a variety of different collection containers.

### Background to the Invention

Cord blood collection is performed for a variety of diagnostic and screening laboratory tests such as, fetal acid-base analysis, blood typing, genetic screening, haemoglobinopathy screening, coagulopathy screening, and sepsis screening. Cord blood collection is also performed for umbilical stem cell harvesting. When feasible, collecting cord blood offers many advantages over peripheral blood sampling in the neonate. These advantages include, but are not limited to, an ability to obtain larger volumes of blood without compromising the neonate, and a reduction in phlebotomy induced anaemia (one of the leading causes of anaemia in pre-term patients).

There are two general methods utilised for cord blood collection. One method involves draining the cord blood from the cut end of an umbilical cord (by milking for example), generating a mixed arterial/venous blood sample. The other involves withdrawing the cord blood directly from the umbilical artery/vein using a needle and syringe. The latter is currently required for reliable cord blood acid-base analysis. However, a wide range of common diagnostic and screening tests (approximately 2000 yearly at the Rotunda Hospital alone) can be completed utilising cord blood obtained via the draining technique. This technique generally consists of "milking" the blood from the distal end of a cut umbilical cord, while still attached to the delivered placenta, into an uncapped universal collection Tube (e.g. 20 ml Sarstedt universal tube). This milking technique involves holding the universal tube in one hand while securing the umbilical cord with the index finger and thumb of the same hand. Simultaneously, the umbilical cord is held with the other hand and cord blood is 'milked' into the universal tube until the required volume of clot-free blood is collected. During this process, blood spillage is common and the exterior of the universal tube is often contaminated with blood and must be cleaned afterwards. Furthermore, personal protective clothing and equipment (PPCE) is required, as blood splashes are common. The universal tube is then sent to the laboratory where the blood must then be manually transferred from the universal tube into a range of standard laboratory tubes containing the appropriate preservative agent (e.g. EDTA).

Alternatively, even in the instance that a direct venous or arterial sample is not required (e.g. blood typing, genetic testing, haemoglobinopathy/coagulopathy screening), a needle and syringe may still be introduced to access localized areas of blood along the umbilical cord. This allows for the blood sample to be directly transferred into the appropriate blood collection tube. Using a needle and syringe can also reduce the amount of blood spillage and contamination encountered when milking the blood into a universal collection tube. When the needle and syringe technique is employed, the following methods can be used:
a) The umbilical cord is 'anchored' by the placenta, *i.e.* the cord is left attached to the placenta to provide an anchoring base. One hand holds the umbilical cord extended away from the placenta and the other holds and inserts the needle to withdraw blood from a visible blood vessel. The blood is then transferred from the syringe to a blood tube.
b) The umbilical cord is wrapped around one hand and the other hand holds and inserts the needle to withdraw the blood from a visible blood vessel. The blood is then transferred from the syringe to a blood tube.
c) Two clamps are attached to a length of the cord and the cord is wrapped around the clamps. The individual's two hands are then free to insert the needle and withdraw the blood. The blood is then transferred from the syringe to a blood tube.

There are several problems associated with the methods of cord blood collection described above. These include:
- Sub-sampling errors associated with transfer from a universal collection tube to the appropriate analysis tube (e.g. incorrect labelling).
- Jeopardized sample viability associated with lack of anti-coagulant factors in the universal collection tube.
- Overall encumbrance of the process.
- Occupational hazard of blood splash and leakage.
- Occupational hazard of needle stick injury.
- Quality control and safety non-conformance with regulatory authorities (Irish National Accreditation Board, European Union *Prevention of Sharps Injuries in the Healthcare Sector Regulations)*

Other products which have been designed to collect cord blood include the Umbilicup™. With Umbilicup™, a mixed arterial/venous sample is drained from a cut cord. Umbilicup™ requires the user to either clamp or clamp and cut the cord. With the Umbilicup™, blood is allowed to pool before it is transferred to an evacuated storage tube via needle. This introduces the risk of blood clot formation, which could jeopardize sample integrity. Furthermore, due to the needle design of the Umbilicup™, the collection tube cap must allow for needle insertion, limiting the range of blood collection tubes that are compatible. Finally, although minimized, the risk of needle stick injury is not eliminated with Umbilicup™.

US Patent 8486034 describes a complex device for extracting blood from a placenta or umbilical cord using a pressurised container and a needle. The risk of needle stick injuries is present and the complexity of the device make it a cumbersome and over-complicated device to use. GB 2302734 A describes a devised with a lid and a needle used for collecting cord blood from a cut and clamped section of an umbilical cord. The risk of needle stick injury is problematic. US Patent 6102871 describes cord blood collection device comprising a funnel with two outlets. A section of cord is cut from the placenta, clamped at one end and placed in the funnel. The device is unwieldly and tubes are loosely fitted to the outlets when collecting blood from the cord. US 2010/081966 describes an umbilical cord cell harvesting device which comprises a ratchet mechanism to stem the flow of blood from a section of umbilical cord. The section of cord is placed within a container having multiple components. US 2003/220601 describes a body fluid collection device which comprises a container in fluid communication with a luer collection device having a sheathed needle. The risk of a needle stick injury is apparent with this device and a section of cord is cut and clamped before being placed in the container. This introduces issues with the cord slipping out and the use of gravity alone to collect blood. There is a risk of blood pooling and thus blood clot formation occurring when using this device (and similar devices above).

It is an object of the present invention to overcome at least some of the above-mentioned problems.

### Summary of the Invention

According to the present invention there is provided a cord blood collection device, the device comprising a chamber in fluid communication with a tapered nozzle; and in which the tapered nozzle further comprises a series of steps along its length, the series of steps configured to engage with a series of collection containers having different diameters.

Preferably, the tapered nozzle 4 comprises a distal end 10, an aperture 12 and an internal channel 14.

Preferably, the step 20 is configured to engage with an open end 106 of the collection container 100.

Preferably, the nozzle 4 further comprises a luer lock 50 at a distal end 10 thereof. Preferably, the luer lock 50 is integrated with and permanently connected to the distal end 10 of the nozzle 4. The luer lock 50 may also be configured to secure to the nozzle 4 by means of a male fitting on the luer lock 50 which screws into threads in the distal end 10 of the nozzle 4. The luer lock 50 may also be configured to secure to the nozzle 4 by means of a female fitting on the luer lock 50 which screw onto a male fitting on the distal end 10 of the nozzle 4.

Preferably, the chamber 2 comprises a wall 8 enclosing a space 7 and an open end 6a adapted to receive a distal end of an umbilical cord and an open end 6b in fluid communication with the nozzle 4.

Preferably, the shape of the chamber 2 is substantially circular or multi-sided. Preferably, the chamber 2 further comprises a stepped portion 16 adapted to receive and engage with the open end 106 of the collection container 100. The stepped portion 16 is located between the open end 6b and the nozzle 4.

Generally, the chamber 2 is between 1cm and 30cm in length, preferably between about 2cm and about 25 cm in length, more preferably between about 3cm and about 20cm in length, and suitably between about 3cm and about 10cm in length. Ideally, the chamber 2 is between about 3 and about 5 cm in length, such as about 4cm in length.

Preferably, the wall 8 of the chamber 2 further comprises a textured internal surface.

In one embodiment of the present invention, the cord blood collection device may be a pre-sterilised, single-use device.

In one embodiment, the cord blood collection device is suitable for multiple uses. Preferably, the device may be suitable for multiple sterilisation cycles in, for example, a central sterile services department (CSSD) in the medical facility.

The cord blood collection device may be thermoformed as a single part, consisting of two main elements: 1) a chamber, which is adapted to accommodate an umbilical cord, and 2) a nozzle, which accommodates and engages with a collection container.

In one embodiment, the material of construction is suitably a durable yet rigid material, for example polypropylene, polyethylene (PE), polyethylene terephthalate (PET), polyethylene terephthalate copolymer (PETG), amorphous polyethylene terephthalate (APET), Acrylonitrile-Butadiene-Styrene (ABS), Styrene Acrylonitrile (SAN), high impact polystyrene (HIPS), polycarbonate (PC), (thin-walled) surgical stainless steel. In another embodiment, the device could also be made of non-medical polymers such as, polystyrene.

In one embodiment, the wall of the chamber has a thickness of between about 0.05mm to about 2.0 mm, preferably about 0.1 mm to about 1.8 mm, more preferably between about 0.75 mm and 1.8 mm, and ideally about 1.3 mm to about 1.6 mm. In one embodiment, the wall of the nozzle has the same or similar thickness to the chamber, that is, about 1.4 mm.

Preferably, the wall of the chamber further comprises a textured internal surface.

Preferably, the device is constructed from non-medical materials such as polystyrene.

Preferably, the device is pre-sterilised. Preferably, the device is a single-use device. Preferably, the device is suitable for multiple uses. Preferably, the device is suitable for multiple sterilisation cycles in, for example, a central sterile services department (CSSD) in a medical facility.

Preferably, the device is needleless.

Disclosed is also a stand for use with the device described above, the stand comprising a base and a vessel. Preferably, the stand further comprises an enclosure configured to secure and contain a cap of a container.

Further disclosed is also a kit for collecting umbilical cord blood, the kit comprising the device as described above. Preferably, the kit further comprises the stand described herein.

Preferably, the kit further comprises one or more collection containers 100. In addition, the kit further comprises a catheter, a connecting tube, a cord clamp, a diathermy/instrument pouch, a drape, a baby blanket, a hand towel, a mayo cover, a sharps box, a swab, a yankauer, a gown, a trolley cover, a wrap, a crepe, a bag and a breather.

Also disclosed is an umbilical cord blood collection nozzle wherein one end is open to receive an umbilical cord and a second end is inwardly tapered and open, and configured to dispense said umbilical cord blood to a collection container.

In one embodiment, the term "cord blood" should be understood to mean blood sourced from the umbilical cord, which contains blood from the umbilical cord itself but also blood remaining in the placenta after childbirth. The cord blood collected contains (haematopoietic) stem cells, which can be used to treat haematopoietic and genetic diseases.

### Brief Description of the Drawings

The invention will be more clearly understood from the following description of an embodiment thereof, given by way of example only, with reference to the accompanying drawings, in which:-
**Figure 1A** illustrates a side view of one embodiment of the cord blood collection device of the present invention; while **Figure 1B** illustrates a bottom view, **Figure 1C** a perspective view, and **Figure 1D** the side view of Figure 1A with a collection container attached thereto.
**Figure 2A** illustrates a longitudinal view of one embodiment of the device; **Figure 2B** illustrates a bottom view of the embodiment of the device; and **Figure 2C** illustrates a perspective view of the embodiment of the device of the present invention.
**Figure 3A** to **3C** illustrate longitudinal sectional views of the cord blood collection device of Figure 2 engaged with collection tubes of varying diameters.
**Figure 4A** illustrates an array of tubes used for cord blood analysis (Vacuette® 9ml K3EDTA, Sarstedt Monovette® 7.5ml EDTA KE, Vacuette® 2.5ml K3EDTA, Sarstedt Monovette® 2.7ml EDTA KE and Sarstedt Monovette® 2ml blood gas containers, BD Vacutainer® K2E (EDTA)6.0 ml, BD Vacutainer® K2E 4.0 ml.
**Figure** 5 illustrates a side view of one embodiment of the cord blood collection device connected to a sample tube used for cord blood collection and with an umbilical cord *in situ* within the housing of the device.
**Figure 6** illustrates a side view of one embodiment of the cord blood collection device of Figure 1 or Figure 2 showing a luer lock connector at the opening of the tapered nozzle.
**Figures 7A** and **7B** illustrate perspective views of a stand for the device of the present invention.

### Detailed Description of the Drawings

### Materials and Methods

When the device described herein is utilised, umbilical cord blood can be safely and effectively collected from the cut end of the cord. The required materials include: cut end of delivered umbilical cord (where the cord is either (a) attached to placenta and cut or (b) cut from placenta and clamped at either or both cut ends), blood collection tube and the device described herein. Personal Protective Clothing and Equipment (PPCE) such as gloves, gowns, face masks *etc.* would also be worn by the person collecting the cord blood, as per the safe work procedures of the institute collecting the blood. The blood collection tube is uncapped and the nozzle of the device described herein is inserted into the tube. A secure connection between the nozzle of the device described herein and the collection tube is achieved via the tapered, stepped design of the nozzle. One hand is used to insert the distal end of the cut cord into the chamber of the device described herein while the other hand holds the device described herein with the collection tube securely attached to it. The distal end of the cord is stabilised in place in the chamber with the thumb of the same hand holding the device and the attached tube. The other hand is then used to milk the cord blood into the device. The blood is directly funnelled into the collection tube. When a sufficient volume of clot-free blood has been collected, the device described herein is released from the blood collection tube and set aside. The blood-filled collection tube can then be placed in a collection tube holder while gloves are changed. The tube is then capped with clean gloves. When using the device described herein as a single-use item, the device can be discarded along with the cord/placenta. Where the device is a re-usable item, the device can be set aside for cleaning and sterilisation and the cord/placenta discarded. The collected blood can then be sent for blood typing, stem cell collection, genetic testing and a range of diagnostic and screening tests.

A number of Dublin maternity hospitals were using a 20ml syringe (with the plunger removed) as a way of funnelling cord blood straight into appropriate laboratory containers but, on testing by clinical staff in the Rotunda Hospital, this method was found to be unsafe and unwieldy.

The present invention provides a cord blood collection device that is safe, practical, efficient and simple to use. The cord blood collection device described herein addresses the safety and sample quality issues associated with a commonly employed method of cord blood collection. The current problems include: jeopardized sample viability due to lack of appropriate preservative agents in universal collection tube, sub-sampling error, risk of blood splash and leakage, risk of needle stick injury and overall encumbrance of the process.

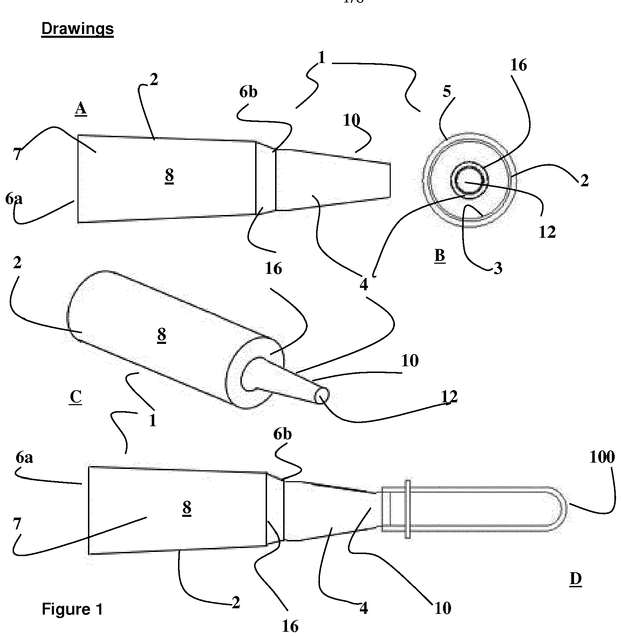

Referring now to the figures, where **Figure 1** illustrates a general embodiment of an umbilical cord blood collection device of the present invention, and is generally referred to by reference numeral 1. The umbilical cord blood collection device 1 comprises a chamber 2 in fluid communication with a tapered nozzle 4. The chamber 2 comprises a wall 8 enclosing a space 7, the wall 8 having an inner diameter 3 and outer diameter 5. The chamber 2 further comprises an open end 6a, adapted to receive and accommodate a distal end of an umbilical cord, and an open end 6b which is in fluid communication with the nozzle 4. The open end 6b is configured to transfer fluid received from the umbilical cord accommodated within the space 7 of the chamber 2 to the nozzle 4. While the chamber 2 can be any desired shape (for example, circular or multi-sided (for example, triangle, square, rectangle, oval, 4-sided, 5-sided, 6-sided or more)), the chamber 2 is generally substantially circular. The chamber 2 has a length of approximately 4 cm, which allows for a sufficient section of cord to be inserted therein, and preventing the cord from slipping out of the device 1. In one embodiment, the wall 8 enclosing space 7 has a textured internal surface, which provides purchase on the distal end of the umbilical cord when accommodated within the device 1. During cord blood collection, the cord is further stabilised within the chamber 2 with the thumb of the hand used to hold the device 1 and an attached collection container 100.

The nozzle 4 comprises a distal end 10 in fluid communication with the open end 6b of the chamber 2 via the channel 14. The distal end 10 has an aperture 12, which is in fluid communication with the open end 6b. The nozzle 4 is tapered, with a larger diameter at the open end 6b when compared to the distal end 10.

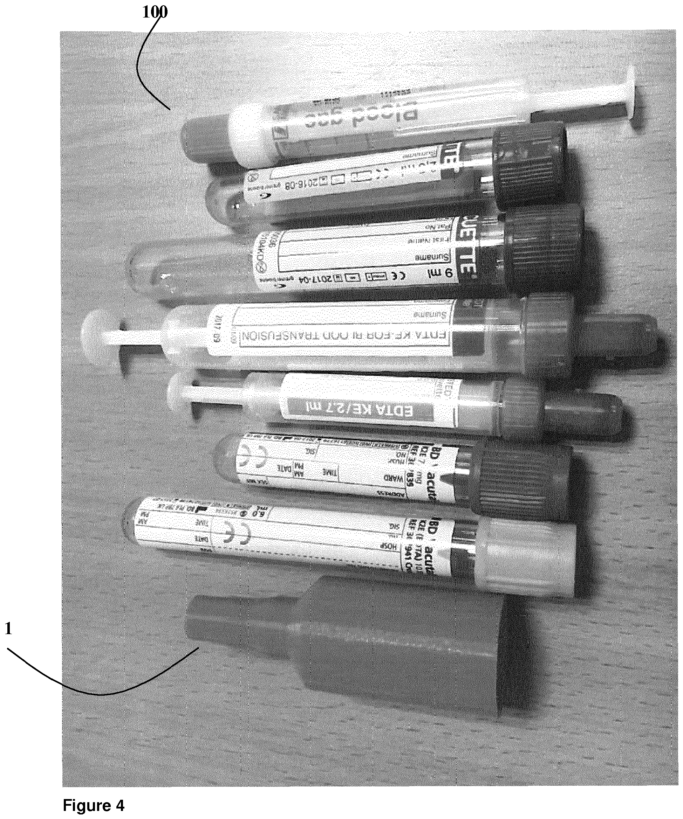

The chamber 2 further comprises a stepped portion 16, which is in fluid communication with, and in between, the open end 6b and nozzle 4. The stepped portion 16 is adapted to receive and (reversibly) engage with the mouth of the collection container 100 having a suitable diameter (see **Figure 4**). In certain scenarios, the diameter of the collection container 100 will be of a particular size that the mouth of the container 100 will not engage with the stepped portion 16 but with the surface of the tapered nozzle 4 itself (see **Figure 1D**). Typically, the collection container 100 comprises a housing 102 having an open end (mouth) 106 and a rim or lip 108 surrounding the open end 106. The engagement between the device 1 and the collection container 100 provides a secure fit. The secure fit is reversible and is achieved by the gradual taper on the nozzle 4 which provides interference fit with the container 100 but would not be so tight so as to hinder the removal of the container 100 that would be filled with cord blood from the device 1. This reversibly secure fit will reduce any risk of blood leakage that would otherwise result in contamination of the exposure of the collection container 100.

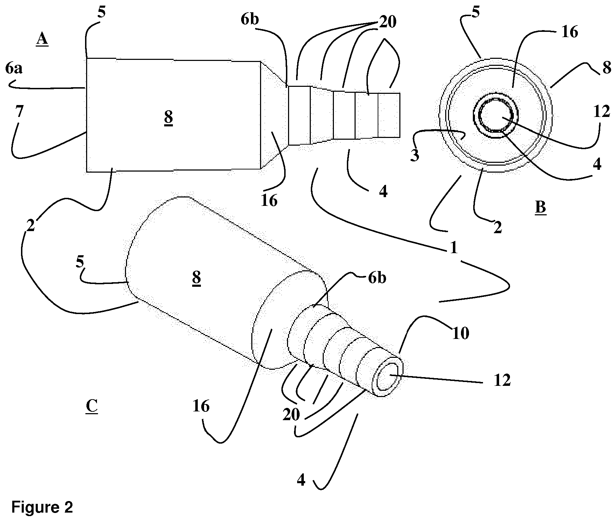

Referring to **Figure 2****,** there is illustrated an embodiment of an umbilical cord blood collection device 1 of the present invention, in which parts described with reference to the previous embodiment are assigned the same numerals. In the embodiment, the nozzle 4 comprises a series of steps 20 that run from the stepped portion 16 to the aperture 12 of the distal end 10. This is more clearly seen in **Figure 2A** and **2C** where the circumference of the steps 20 start off widest at the stepped portion 16 and progressively narrows towards the aperture 12 of the distal end 10 of the nozzle 4.

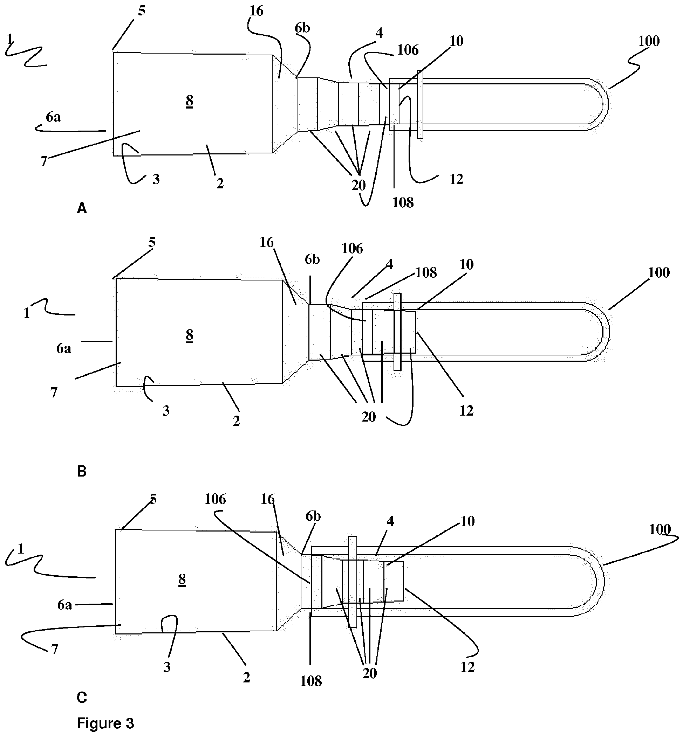

The steps 20 are adapted to securely fit to, and reversibly release from, the open end 106 of the collection container 100 having a particular diameter housing 102 (see **Figure 3A-C**), as described above for Figure 1. The series of steps 20 are configured to accommodate collection containers 100 of varying diameters, such as a Vacuette® 9ml K3EDTA, a Sarstedt Monovette® 7.5ml EDTA KE, a Vacuette® 2.5ml K3EDTA, a Sarstedt Monovette® 2.7ml EDTA KE, a Sarstedt Monovette® 2ml blood gas containers, a BD Vacutainer® K2E (EDTA)6.0 ml, a BD Vacutainer® K2E 4.0 ml. (see **Figure 4**). This versatility in being able to connect to and fit a wide variety of collection containers 100 is one advantage of the device 1 of the invention.

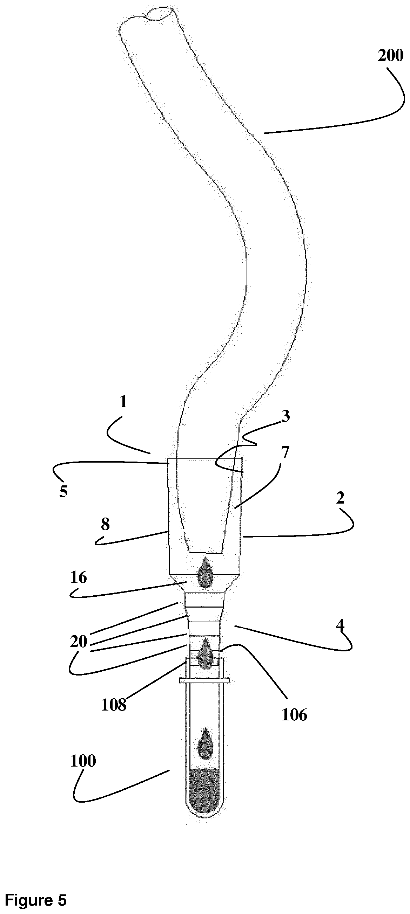

In use, as illustrated in **Figure 5****,** the open end 106 of the collection container 100 is placed over the nozzle 4 of the device 1. The aperture 12 is positioned within the housing 102 of the collection container 100 and the rim 108 of the open end 106 of the collection container 100 engages with either one of the steps 20 or the stepped portion 16. The diameter of the open end 106 of the container 100 will determine where along the nozzle 4, such as which step 20 or the stepped portion 16, the container 100 engages. The collection container 100 generally finds a position on the nozzle 4 or stepped portion 16 so that the container 100 fits to the device 1 but not so tight as to make it difficult to remove when the cord blood has been collected. A distal end of an umbilical cord 200 is placed through the open end 6a of the chamber 2 and into the space 7 such that it is enclosed by the wall 8. Any blood or fluid removed from the umbilical cord 200 either by physical manipulation or gravity will flow from the open end 6b of the chamber 2 and into the nozzle 4. The fluid or blood will be directed via the internal channel 14 of the nozzle 4, through the aperture 12 and into the collection container 100.

The device 1 is designed to accept the distal end of an umbilical cord and securely connect with a range of collection containers (100), enabling the safe and efficient collection of cord blood. It is used on delivered umbilical cords/placentas. Sterile devices can be used in the immediate patient care area. This would allow the device to be incorporated, for example, in sterile/surgical packs used in operating theatres. A caesarean section pack for example, would typically include the device 1 of the present invention, a catheter, a connecting tube, a cord clamp, a diathermy/instrument pouch, a drape, a baby blanket, a hand towel, a mayo cover, a sharps box, a swab, a yankauer, a gown, a trolley cover, a wrap, a crepe, a bag and a breather.

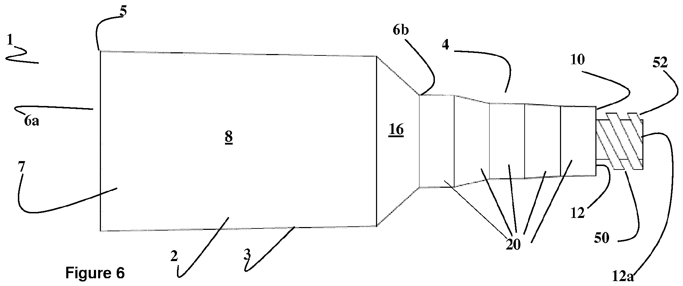

Referring to **Figure 6****,** there is illustrated an embodiment of an umbilical cord blood collection device 1 of the present invention, in which parts described with reference to the previous embodiment are assigned the same numerals. In the embodiment, the nozzle 4 comprises the series of steps 20 that run from the stepped portion 16 to the aperture 12 of the distal end 10. The circumference of the steps 20 start off widest at the stepped portion 16 and progressively narrows towards the aperture 12 of the distal end 10 of the nozzle 4. The nozzle 4 further comprises a luer lock 50 in fluid communication with the aperture 12 of the distal end 10 via a channel within the lock 50 which ends with an aperture 12a distal the aperture 12. The luer lock 50 further comprises threads 52 which are configured to engage with a tabbed hub on a female fitting which screws onto the threads 52 on the lock 50. The channel within the luer lock 50 can also have an internal taper (wider towards aperture 12a and tapering towards the distal end 10) to engage with a male fitting, such as a needless syringe. The luer lock 50 incorporated into device 1 allows for the collection of cord blood when engaged with the current collection tubes 100 of varying diameters. The luer lock 50 connection expands the possible range/number of devices/collection systems that may be used now and in the future for example; collection tubes to bags, syringes, interconnectors *etc.*

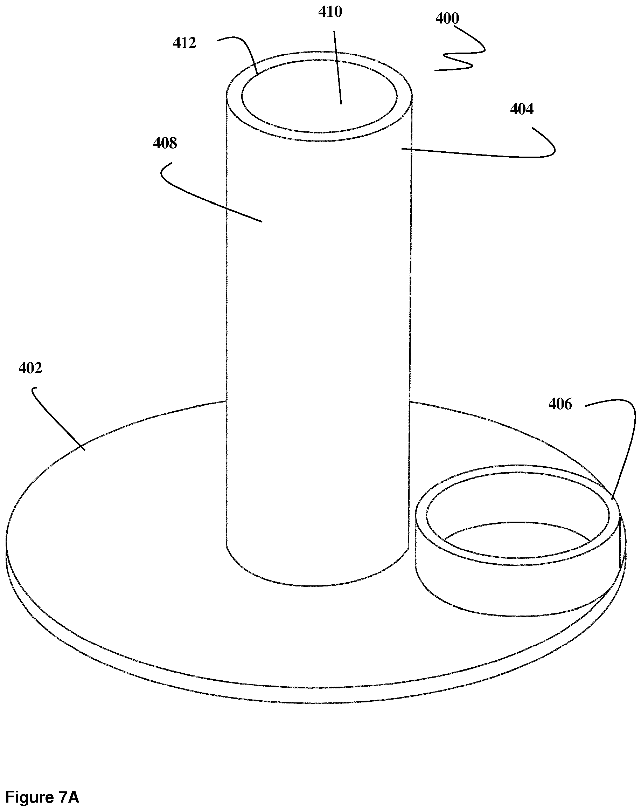

Referring to **Figure 7A** there is illustrated a stand 400 for use with the device 1 described herein. The stand 400 comprises a base 402 upon which is a vessel 404 and an enclosure 406. The vessel 404 is generally a cylinder having an outer wall 408 defining a lumen 410, an open end 412 and a closed end distal the open end where the outer wall 408 contacts the base 402. The vessel 404 is configured to accept and support the collection container 100, while the enclosure 406 is configured to hold and secure a cap of the container 100 when the container 100 is engaged with the device 1.

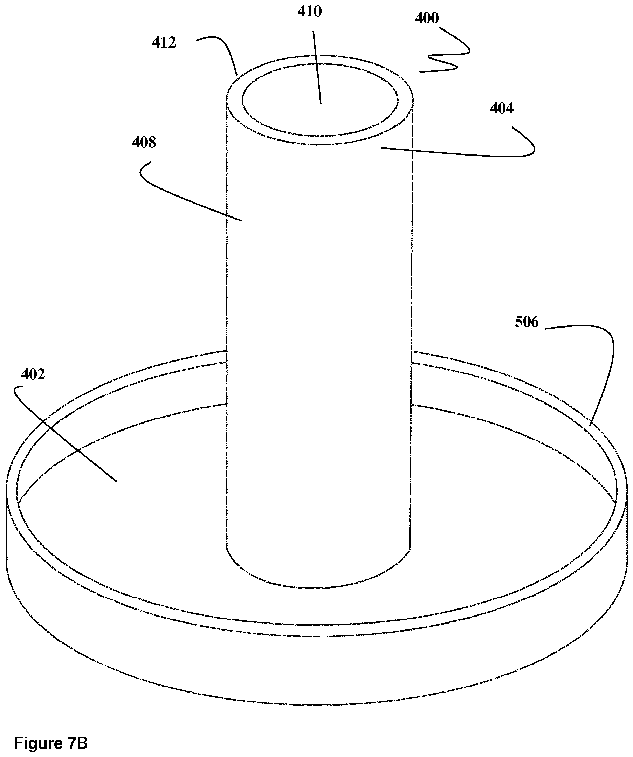

Referring to **Figure 7B****,** there is illustrated a stand 400 for use with the device 1 described herein in which parts or steps described with reference to the **Figure 7A** are assigned the same numerals. In **Figure 7B****,** the enclosure 406 of Figure 7A is replaced with an enclosure 506. The enclosure 506 forms a barrier around the outer edge of the base 402 and creates a moat-like structure surrounding the vessel 404. The enclosure 506 permits the user to place the cap of the container 100 when the container 100 is engaged with the device 1. In some instances, the enclosures 406,506 could be combined on the same base 402, with the enclosure 406 within the confines of the enclosure 506.

In use, the container 100 is placed in the vessel 404 and the cap is removed from the container 100 and placed in the enclosure 406,506. The nozzle 4 of the device 1 is placed within the open end 106 of the collection container 100 and the cord blood is collected as described for **Figure 5** above. The advantage of using the stand 400 is that there is a greatly reduced likelihood of contaminating collection container 100 and its label as the operators gloved hands can exclusively now hold device 1 when collecting cord blood. Stand 400 eliminates the need for a variety of collection container holders (determined by the specific collection container used), as stand 400 fits all current commonly used collection containers.

Some of the advantages of the device 1 of the present invention are that:
(a) It allows for a universal and precise fit applicable to the most commonly used laboratory blood or fluid collection containers, such as, Vacuette® 9ml K3EDTA, BD Vacutainer® K2E (EDTA) 6.0 ml, BD Vacutainer® K2E 4.0 ml, Sarstedt Monovette® 7.5ml EDTA KE, Vacuette® 2.5ml K3EDTA, Sarstedt Monovette® 2.7ml EDTA KE and Sarstedt Monovette® 2ml blood gas containers. As such, blood is collected directly into the collection tube containing the appropriate preservative agent. This helps minimize jeopardized sample viability (e.g. due to clotting).
(b) Precision fit with the appropriate blood tubes reduces the risk of blood leakage and spillage during the milking process. This in turn reduces the occupational risk of blood exposure.
(c) Direct funnelling into the appropriate blood tube eliminates the need for additional sub-sampling during laboratory analysis. This avoids sub-sampling errors.
(d) The device 1 does not require a needle for blood collection or transfer. The blood is sampled directly from the umbilical cord into the appropriate collection container. This avoids any needle stick injuries.
(e) The chamber 2 which houses the distal end of the umbilical cord, is designed so that the cord can be easily inserted and held in the device 1. This is achieved by simply inserting the cord into the chamber 2 while stabilizing the cord against the wall 8 of the chamber 2 with the one's thumb. The chamber length of approximately 4 cm allows for a sufficient section of cord to be inserted, preventing the cord from slipping out. Furthermore, the chamber's inner surface is textured, providing purchase on the distal end of the umbilical cord. The texture would also allow clearance for blood flow between the surface of the cut cord and the chambers inner surface.
(f) The use of the nozzle 4 allows for the blood to be directly transferred into the collection container without pooling during transfer. This helps minimize jeopardized sample viability due to clotting.
(g) While other methods of cord blood collection can be cumbersome and may require two sets of hands, the device 1 described herein can be easily used by one individual using both hands. Furthermore, the use of stand 400 means any current/common collection container 100 and cap will be securely accommodated.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A cord blood collection device (1), the device (1) comprising a chamber (2) in fluid communication with a tapered nozzle (4); and in which the tapered nozzle (4) further comprises a series of steps (20) along its length, the series of steps (20) configured to engage with a series of collection containers (100) having different diameters.

2. A device (1) according to Claim 1, wherein the tapered nozzle (4) comprises a distal end (10), an aperture (12) and an internal channel (14).

3. A device (1) according to Claim 1 wherein one of the series of steps (20) is configured to engage with an open end (106) of one of the series of collection containers (100).

4. A device (1) according to any one of the preceding Claims, wherein the nozzle (4) further comprises a luer lock (50) at a distal end (10) thereof.

5. A device (1) according to Claim 4, wherein the luer lock (50) is integrated with and permanently connected to the distal end (10) of the nozzle (4).

6. A device (1) according to Claim 4, wherein the luer lock (50) is configured to secure to the nozzle (4) by means of a male fitting on the luer lock (50) which screws into threads in the distal end (10) of the nozzle (4).

7. A device (1) according to Claim 4, wherein the luer lock (50) is configured to secure to the nozzle (4) by means of a female fitting on the luer lock (50) which screw onto a male fitting on the distal end (10) of the nozzle (4).

8. A device according to any one of the preceding Claims, wherein the chamber (2) comprises a wall (8) enclosing a space (7) and an open end (6a) adapted to receive a distal end of an umbilical cord and an open end (6b) in fluid communication with the nozzle (4).

9. A device (1) according to any one of the preceding claims, wherein the chamber (2) further comprises a stepped portion (16) adapted to receive and engage with the open end (106) one of the series of collection containers (100).

10. A device (1) according to any one of the preceding claims, wherein the wall (8) of the chamber (2) further comprises a textured internal surface.

11. A device (1) according to any one of the preceding claims, wherein the device is constructed from a durable yet rigid material selected from polypropylene, polyethylene (PE), polyethylene terephthalate (PET), polyethylene terephthalate copolymer (PETG), amorphous polyethylene terephthalate (APET), Acrylonitrile-Butadiene-Styrene (ABS), Styrene Acrylonitrile (SAN), high impact polystyrene (HIPS), polycarbonate (PC), surgical stainless steel.

12. A device (1) according to any one of Claims 1 to 10, where the device is constructed from polystyrene.

13. A device (1) according to any one of the preceding claims, wherein the device is pre-sterilised.

## Patentansprüche

1. Nabelschnurblut-Sammelvorrichtung (1), wobei die Vorrichtung (1) eine Kammer (2) in Flüssigkeitsverbindung mit einer konischen Düse (4) umfasst; und in der die konische Düse (4) weiter eine Reihe von Stufen (20) entlang ihrer Länge umfasst, wobei die Reihe von Stufen (20) zum Eingreifen in eine Reihe von Sammelbehältern (100), die unterschiedliche Durchmesser aufweisen, konfiguriert ist.

2. Vorrichtung (1) nach Anspruch 1, wobei die konische Düse (4) ein distales Ende (10), eine Öffnung (12) und einen inneren Kanal (14) umfasst.

3. Vorrichtung (1) nach Anspruch 1, wobei eine der Reihe von Stufen (20) zum Eingreifen in ein offenes Ende (106) von einem der Reihe von Sammelbehältern (100) konfiguriert ist.

4. Vorrichtung (1) nach einem der vorstehenden Ansprüche, wobei die Düse (4) weiter einen Luer-Lock-Anschluss (50) an einem distalen Ende (10) davon umfasst.

5. Vorrichtung (1) nach Anspruch 4, wobei der Luer-Lock-Anschluss (50) mit dem distalen Ende (10) der Düse (4) integriert und permanent verbunden ist.

6. Vorrichtung (1) nach Anspruch 4, wobei der Luer-Lock-Anschluss (50) zur Sicherung an der Düse (4) mithilfe eines männlichen Verbindungsstücks an dem Luer-Lock-Anschluss (50) konfiguriert ist, das in das Gewinde in dem distalen Ende (10) der Düse (4) geschraubt wird.

7. Vorrichtung (1) nach Anspruch 4, wobei der Luer-Lock-Anschluss (50) zur Sicherung an der Düse (4) mithilfe eines weiblichen Verbindungsstücks an dem Luer-Lock-Anschluss (50) konfiguriert ist, das auf ein männliches Verbindungsstück an dem distalen Ende (10) der Düse (4) geschraubt wird.

8. Vorrichtung (1) nach einem der vorstehenden Ansprüche, wobei die Kammer (2) Folgendes umfasst: eine Wand (8), die einen Raum (7) umschließt, und ein offenes Ende (6a), das zur Aufnahme eines distalen Endes einer Nabelschnur geeignet ist, und ein offenes Ende (6b) in Flüssigkeitsverbindung mit der Düse (4).

9. Vorrichtung (1) nach einem der vorstehenden Ansprüche, wobei die Kammer (2) weiter einen abgestuften Teil (16) umfasst, der zur Aufnahme und zum Eingreifen in das offene Ende (106) von einem der Reihe von Sammelbehältern (100) geeignet ist.

10. Vorrichtung (1) nach einem der vorstehenden Ansprüche, wobei die Wand (8) der Kammer (2) weiter eine strukturierte innere Oberfläche umfasst.

11. Vorrichtung (1) nach einem der vorstehenden Ansprüche, wobei die Vorrichtung aus einem strapazierfähigen, doch steifen Material konstruiert ist, das aus Folgenden ausgewählt ist: Polypropylen, Polyethylen (PE), Polyethylenterephthalat (PET), Polyethylenterephthalat-Copolymer (PETG), amorphem Polyethylenterephthalat (APET), Acrylnitril-Butadien-Styrol (ABS), Styrol-Acrylnitril (SAN), hochschlagzähem Polystyrol (HIPS), Polycarbonat (PC), chirurgischem Edelstahl.

12. Vorrichtung (1) nach einem der Ansprüche 1 bis 10, wo die Vorrichtung aus Polystyrol konstruiert ist.

13. Vorrichtung (1) nach einem der vorstehenden Ansprüche, wobei die Vorrichtung vorsterilisiert ist.

## Revendications

1. Dispositif de prélèvement de sang de cordon (1), le dispositif (1) comprenant une chambre (2) en communication fluidique avec une buse conique (4) ; et dans lequel la buse conique (4) comprend en outre une série de crans (20) le long de sa longueur, la série de crans (20) configurée pour entrer en prise avec une série de contenants de prélèvement (100) ayant des diamètres différents.

2. Dispositif (1) selon la revendication 1, dans lequel la buse conique (4) comprend une extrémité distale (10), une ouverture (12) et un canal interne (14).

3. Dispositif (1) selon la revendication 1, dans lequel un cran de la série de crans (20) est configuré pour entrer en prise avec une extrémité ouverte (106) d'un contenant de la série de contenants de prélèvement (100).

4. Dispositif (1) selon l'une quelconque des revendications précédentes, dans lequel la buse (4) comprend en outre un verrouillage Luer (50) au niveau d'une extrémité distale (10) de celle-ci.

5. Dispositif (1) selon la revendication 4, dans lequel le verrouillage Luer (50) est d'un seul tenant et connecté de manière permanente avec l'extrémité distale (10) de la buse (4).

6. Dispositif (1) selon la revendication 4, dans lequel le verrouillage Luer (50) est configuré pour se fixer à la buse (4) au moyen d'un raccord mâle sur le verrouillage Luer (50) qui se visse dans des filetages dans l'extrémité distale (10) de la buse (4).

7. Dispositif (1) selon la revendication 4, dans lequel le verrouillage Luer (50) est configuré pour se fixer à la buse (4) au moyen d'un raccord femelle sur le verrouillage Luer (50) qui se visse sur un raccord mâle sur l'extrémité distale (10) de la buse (4).

8. Dispositif selon l'une quelconque des revendications précédentes, dans lequel la chambre (2) comprend une paroi (8) enfermant un espace (7) et une extrémité ouverte (6a) conçue pour recevoir une extrémité distale d'un cordon ombilical et une extrémité ouverte (6b) en communication fluidique avec la buse (4).

9. Dispositif (1) selon l'une quelconque des revendications précédentes, dans lequel la chambre (2) comprend en outre une partie crantée (16) conçue pour recevoir et entrer en prise avec l'extrémité ouverte (106) d'un contenant de la série de contenants de prélèvement (100).

10. Dispositif (1) selon l'une quelconque des revendications précédentes, dans lequel la paroi (8) de la chambre (2) comprend en outre une surface interne texturée.

11. Dispositif (1) selon l'une quelconque des revendications précédentes, dans lequel le dispositif est construit à partir d'un matériau durable mais rigide choisi parmi le polypropylène, le polyéthylène (PE), le poly(téréphtalate d'éthylène) (PET), un copolymère de poly(téréphtalate d'éthylène) (PETG), le poly(téréphtalate d'éthylène) amorphe (PETA), l'acrylonitrile-butadiène-styrène (ABS), le styrène-acrylonitrile (SAN), le polystyrène à fort impact (HIPS), le polycarbonate (PC), l'acier inoxydable chirurgical.

12. Dispositif (1) selon l'une quelconque des revendications 1 à 10, où le dispositif est construit à partir de polystyrène.

13. Dispositif (1) selon l'une quelconque des revendications précédentes, dans lequel le dispositif est préstérilisé.
